(19) Europäisches Patentamt
European Patent Office
Office européen des brevets

(11) **EP 4 195 213 A1**

(12) **EUROPEAN PATENT APPLICATION**
published in accordance with Art. 153(4) EPC

(43) Date of publication:
**14.06.2023 Bulletin 2023/24**

(21) Application number: **22747568.8**

(22) Date of filing: **21.03.2022**

(51) International Patent Classification (IPC):
*G16H 20/00* (2018.01)   *G16H 10/60* (2018.01)
*G16H 50/20* (2018.01)   *A61B 5/00* (2006.01)

(86) International application number:
**PCT/KR2022/003881**

(87) International publication number:
**WO 2023/068462 (27.04.2023 Gazette 2023/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:   **22.10.2021   KR 20210141738**

(71) Applicant: **Welt Corp., Ltd
Seoul 06628 (KR)**

(72) Inventors:
• **KANG, Sung Ji**
  **Seoul 06366 (KR)**
• **ROH, Hye Gang**
  **Seoul 05507 (KR)**
• **JUNG, Hwa Young**
  **Gyeonggi-do 12249 (KR)**
• **LEE, Danny Onsung**
  **Seoul 04091 (KR)**
• **KIM, Hye Ryung**
  **Gyeonggi-do 12997 (KR)**

(74) Representative: **AWA Sweden AB
Box 5117
200 71 Malmö (SE)**

(54) **DATA-BASED SLEEP DISORDER TREATMENT METHOD, AND APPARATUS PERFORMING METHOD**

(57)   Provided is a method of treating a sleep disorder based on data and an apparatus for performing the method. The method of treating a sleep disorder based on data includes determining, by a sleep disorder treatment apparatus, sleep quality, determining, by the sleep disorder treatment apparatus, user compliance with a first recommended time in bed (R-TIB), and determining, by the sleep disorder treatment apparatus, a second R-TIB on the basis of the sleep quality and the user compliance, wherein the first R-TIB is a sleep time recommended to the user earlier, and the second R-TIB is a sleep time recommended to the user later.

FIG. 1

```
SLEEP DISORDER
TREATMENT APPARATUS

SLEEP QUALITY DETERMINER
(100)

SLEEP TIME DETERMINER
(110)

USER COMPLIANCE DETERMINER
(120)

R-TIB DETERMINER
(130)

PROCESSOR
(140)
```

EP 4 195 213 A1

**Description**

CROSS-REFERENCE TO RELATED APPLICATION

This application claims priority to and the benefit of Korean Patent Application No. 2021-0141738, filed on October 22, 2021, the disclosure of which is incorporated herein by reference in its entirety.

BACKGROUND

**1. Field of the Invention**

The present invention relates to a method of treating a sleep disorder based on data and an apparatus for performing the method. More particularly, the present invention relates to a method of treating a sleep disorder based on data for treating a sleep disorder of a user based on a sleep efficiency determined using data of the user, and an apparatus for performing the method.

**2. Discussion of Related Art**

With the development of various smart technologies, data regarding personal daily activities is recorded and personal lives are managed more efficiently based on the recorded data. Above all, as interest in health increases, health-related data logging is getting attention. Many users are generating and utilizing various types of data related to the health, such as exercise, diet, and sleep of users, through user devices, such as smartphones and wearable devices. That is, users have started to generate and manage their own health-related data through user devices, such as smartphones or wearable devices, departing from health-related data being generated and managed only by medical institutions in the past.

Health-related data logging is generally performed through wearable devices. A wearable device is a user device carried by a user or attached to a user's body. With the development of the Internet of Things, wearable devices are being widely used to collect health-related data. A wearable device can collect body change information of a user and data on the environment surrounding a user through the device, and can provide advice required for the health of the user based on the collected data.

Currently, a procedure of providing feedback using health-related data acquired through a wearable device is not sophisticated, and thus is not utilized for detailed medical practices. However, according to the development of not only wearable devices but also user devices capable of collecting various types of health-related data, and the refinement of decision algorithms based on health-related data acquired through user devices, health-related data acquired through user devices can be used in actual medical practices.

In particular, performing a behavioral treatment based on sleep data is an area where it can be implemented based on an algorithm, and such digital treatment is being studied and developed in detail.

SUMMARY OF THE INVENTION

The present invention is directed to providing a method of treating a sleep disorder based on data and an apparatus for performing the method that are capable of treating a sleep disorder of a user by recommending a more therapeutically effective bedtime in consideration of the sleep efficiency of the user using data.

The present invention is also directed to providing a method of treating a sleep disorder based on data and an apparatus for performing the method that are capable of treating a sleep disorder of a user by appropriately adjusting a recommended bedtime of the user to control the balance between the patient's compliance and therapeutic performance.

The present invention is also directed to providing a method of treating a sleep disorder based on data and an apparatus for performing the method that are capable of treating a sleep disorder of a user through adaptive management of sleep data and generation of a recommended time in bed (R-TIB) tree in consideration of the user compliance of the user.

The technical objectives of the present invention are not limited to the above, and other objectives may become apparent to those of ordinary skill in the art based on the following descriptions.

Representative configurations of the present invention for achieving the above object are as follows.

According to an aspect of the present invention, there is provided a method of treating a sleep disorder based on data, the method including: determining, by a sleep disorder treatment apparatus, sleep quality; determining, by the sleep disorder treatment apparatus, user compliance with a first R-TIB; and determining, by the sleep disorder treatment apparatus, a second R-TIB on the basis of the sleep quality and the user compliance, wherein the first R-TIB is a sleep time recommended to a user earlier, and the second R-TIB is a sleep time recommended to the user later.

The sleep quality may include sleep efficiency and a sleep deficit, and the user compliance may be determined based on slopes that are set to be different according to ranges of a TIB.

The second R-TIB may be determined based on a user sleep efficiency range, a user sleep deficit range, the user compliance, and a difference in size between the first R-TIB and the TIB, the user sleep efficiency range may be a sleep efficiency range of the user among a (here, a is a natural number) set sleep efficiency ranges based on a first determination on the sleep efficiency (a sleep efficiency determination), the user sleep deficit range may be a user sleep deficit range of the user among b (here, b is a natural number) set sleep deficit ranges based on a second determination on the sleep deficit (a sleep deficit determination), the user sleep deficit range may be determined, after determination of the user sleep efficiency range, based on the determined user sleep efficiency range, and the second R-TIB may be determined based on a default R-TIB window or an increase R-TIB window determined in consideration of a sleep fluctuation range, wherein the increase R-TIB window may be provided such that the sleep fluctuation range is less than a threshold fluctuation range.

According to another aspect of the present invention, there is provided a sleep disorder treatment apparatus for performing a sleep disorder treatment based on data, the sleep disorder treatment apparatus including: a sleep quality determiner configured to determine sleep quality; a user compliance determiner configured to determine user compliance with a first R-TIB; and an R-TIB determiner configured to determine a second R-TIB on the basis of the sleep quality and the user compliance, wherein the first R-TIB is a sleep time recommended to a user earlier, and the second R-TIB is a sleep time recommended to the user later.

The sleep quality may include sleep efficiency and a sleep deficit, and the user compliance may be determined based on slopes that are set to be different according to ranges of a time in bed (TIB).

The second R-TIB may be determined based on a user sleep efficiency range, a user sleep deficit range, the user compliance, and a difference in size between the first R-TIB and the TIB, the user sleep efficiency range may be a sleep efficiency range of the user among a (here, a is a natural number) set sleep efficiency ranges based on a first determination on the sleep efficiency (a sleep efficiency determination), the user sleep deficit range may be a user sleep deficit range of the user among b (here, b is a natural number) set sleep deficit ranges based on a second determination on the sleep deficit (a sleep deficit determination), the user sleep deficit range may be determined, after determination of the user sleep efficiency range, based on the determined user sleep efficiency range, and the second R-TIB may be determined based on a default R-TIB window or an increase R-TIB window determined in consideration of a sleep fluctuation range, wherein the increase R-TIB window may be provided such that the sleep fluctuation range is less than a threshold fluctuation range.

BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:

FIG. 1 is a conceptual diagram illustrating an apparatus for treating a sleep disorder according to an embodiment of the present invention;

FIGS. 2A and 2B are conceptual diagrams illustrating an operation of a user compliance determiner according to an embodiment of the present invention;

FIG. 3 is a conceptual diagram illustrating an operation of a recommended time in bed (R-TIB) determiner according to an embodiment of the present invention;

FIG. 4 is a conceptual diagram illustrating a method of extracting data for determining an R-TIB according to an embodiment of the present invention;

FIG. 5 is a conceptual diagram illustrating a method of processing missing data according to an embodiment of the present invention; and

FIG. 6 is a conceptual diagram illustrating a method of determining an R-TIB according to an embodiment of the present invention.

DETAILED DESCRIPTION OF EXEMPLARY EMBODIMENTS

In the following detailed description, reference is made to the accompanying drawings that show, by way of illustration, specific embodiments in which the invention may be practiced. These embodiments are described in sufficient detail to enable those skilled in the art to practice the invention. It is to be understood that the various embodiments of the invention, although different, are not necessarily mutually exclusive. For example, a certain feature, structure, or characteristic described herein in connection with one embodiment may be implemented within other embodiments without departing from the spirit and scope of the invention. In addition, it is to be understood that the location or arrangement of individual elements within each disclosed embodiment may be modified without departing from the spirit and scope of the invention. The following detailed description is, therefore, not to be taken in a limiting sense, and the scope of the present invention is defined only by the appended claims, appropriately interpreted, along with the full range of equivalents to which the claims are entitled. In the drawings, like numerals refer to the same or similar functionality throughout the several views.

Hereinafter, various exemplary embodiments of the present invention will be described in detail with reference to the accompanying drawings in order to enable those skilled in the art to easily practice the present invention.

Sleep restriction therapy is a treatment technique belonging to cognitive behavioral therapy for insomnia. Sleep restriction therapy is a behavioral treatment of limiting the time in bed (TIB). Because sleep cannot be forcefully restricted, sleep restriction therapy is performed by recommending a time in bed, and the term recommended TIB (hereinafter, R-TIB) is used for this time in bed.

Sleep restriction therapy may help treat insomnia by three mechanisms. The three mechanisms are 1) preventing staying awake in bed, 2) normalizing a sleep pattern, and 3) strengthening a connection between sleep and bed.

Research on sleep restriction therapy conducted up to date is quite limited. Although sleep restriction therapy in its initial version has been applied to cognitive behavioral treatment for insomnia, there are still many studies in progress, such as the operation mechanisms of sleep restriction therapy and the specific methods of sleep restriction therapy.

According to a method of treating a sleep disorder based on data and an apparatus for performing the method according to an embodiment of the present invention, an advanced R-TIB algorithm based on sleep efficiency is disclosed. The aim of the advanced R-TIB algorithm is to determine a more therapeutically effective R-TIB based on various pieces of information, such as user information and environmental information, to control the balance between user compliance and therapeutic performance.

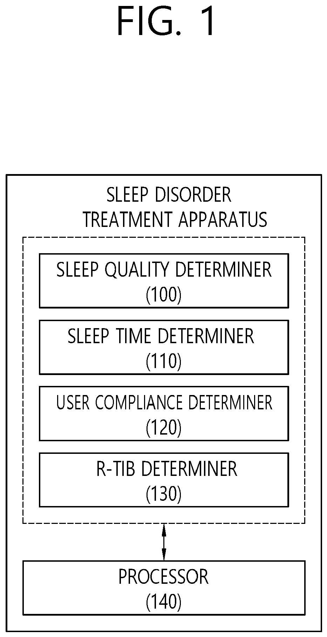

FIG. 1 is a conceptual diagram illustrating an apparatus for treating a sleep disorder according to an embodiment of the present invention.

In FIG. 1, a method of adaptively providing a user with an R-TIB for treatment of a user's sleep disorder is disclosed.

Referring to FIG. 1, a sleep disorder treatment apparatus may include a sleep quality determiner 100, a sleep time determiner 110, a user compliance determiner 120, a recommended time in bed (R-TIB) determiner 130, and a processor 140.

The sleep quality determiner 100 may be implemented to determine sleep quality. The sleep quality involves whether a user has a good sleep state. The sleep quality determiner 100 may determine the sleep quality based on subjective evaluation data and/or objective evaluation data (respiration, brain waves, movement, etc.).

The sleep quality determiner 100 may be implemented to perform a numerical determination on sleep efficiency and whether there is a sleep deficit, to determine a section corresponding to the user in a first determination section (a sleep efficiency determination section) and a second determination section (a sleep deficit determination) for determining an R-TIB, which will be described below.

The sleep time determiner 110 may be implemented to determine a sleep time of the user. The sleep time may be a time that the user has slept in bed and may be determined based on subjective evaluation data and/or objective evaluation data (respiration, brain waves, movement, etc.).

The user compliance determiner 120 may be the degree to which the user complies with a determined R-TIB. The user compliance may also be referred to as R-TIB compliance in another expression. Based on the relationship between an R-TIB determined by the R-TIB determiner 130 and a TIB, which is the actual sleep time of the user, the degree to which the user complies with the determined R-TIB may be determined as the user compliance. In the present invention, measurement of the user compliance may be performed in a linear manner, and details of the method of determining the user compliance will be described below.

The R-TIB determiner 130 may be implemented to determine an R-TIB. The R-TIB may be determined in consideration of the sleep quality of the user, the actual sleep time (TIB) of the user, and the user compliance.

The processor 140 may be implemented to control the operations of the sleep quality determiner 100, the sleep time determiner 110, the user compliance determiner 120, and the R-TIB determiner 130.

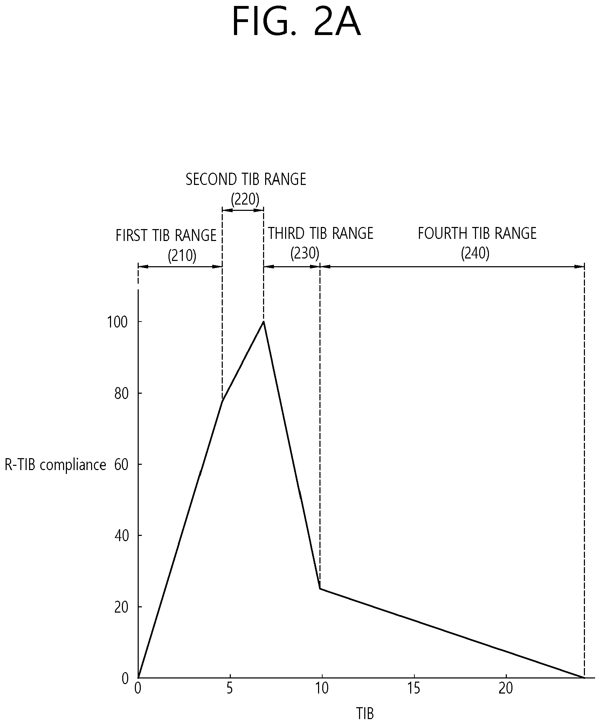

FIG. 2A is a conceptual diagram illustrating an operation of a user compliance determiner according to an embodiment of the present invention.

In FIG. 2A, a method of the user compliance determiner 120 determining user compliance is disclosed.

Referring to FIG. 2A, the user compliance may be determined to have a different slope according to a range of the TIB. A method of determining the user compliance based on the different slope according to the range of the TIB is disclosed below. The ranges of the TIB and the slope values are exemplary values and may be adaptively changed based on a feedback result.

For example, the user compliance (R-TIB compliance) may be calculated as in Equation 1 below.

[Equation 1-1]

$$y = \{0 < x < 4.5 : \frac{\left(100 - s_1(a - 4.5)\right)}{4.5} x$$

$$4.5 \leq x \leq a : s_1 x + 100 - s_1 a$$

$$a < x \leq a + 3 : s_2 x + 100 - s_2 a$$

$$a + 3 < x < M : \frac{(-3 s_2 - 100)}{M - a - 3} x + M * \frac{(3 s_2 + 100)}{M - a - 3}\}$$

Variables in Equation 1-1 denote the following.

y = R-TIB compliance (user compliance)
x = TIB (an actual sleep time of the user after determination of an R-TIB)
a = R-TIB (a recommended sleep time recommended to the user through the R-TIB determiner)
M = Max TIB duration (the maximum time the user can lie in bed)
s1 = slope 1 for calculating the R-TIB compliance (the user compliance)
s2 = slope 2 for calculating the R-TIB compliance (the user compliance)

Here, the numeric values, such as M (e.g., 24), s1, s2, and a+3 (3 is one of variables), may be adaptively changed according to a user compliance feedback result, and a treatment effect.

Specifically, a first TIB range 210 and a second TIB range 220 may be set until the TIB matches the R-TIB without exceeding the R-TIB such that the user compliance in the first TIB range 210 increases relatively rapidly with a larger slope than that in the second TIB range 220.

When the TIB exceeds the R-TIB, a third TIB range 230 and a fourth TIB range 240 may be set such that the user compliance in the third TIB range 230 decreases relatively slowly with a larger slope than that in the fourth TIB range 240.

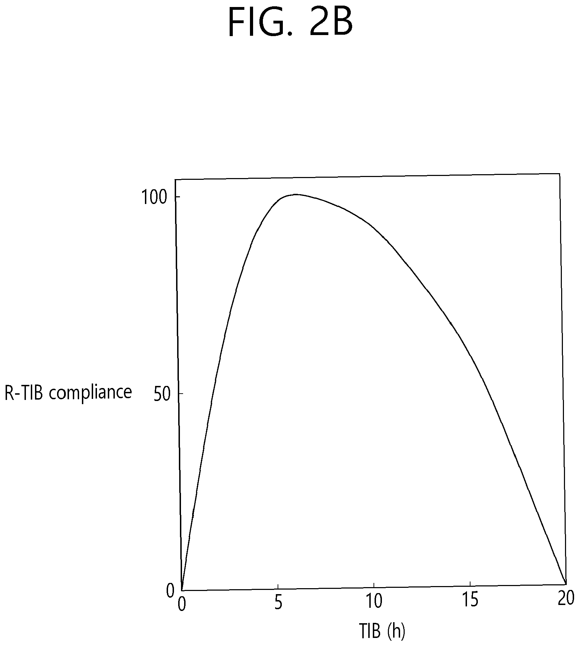

FIG. 2B is a conceptual diagram illustrating an operation of the user compliance determiner according to the embodiment of the present invention.

In FIG. 2B, a method of the user compliance determiner 120 determining the user compliance is disclosed.

Referring to FIG. 2B, the user compliance according to the TIB may be determined based on a user compliance curve which is not linear but parabolic.

In order to determine the user compliance, a piecewise function may be used.

A minimum TIB (e.g., 0 hours) may be set, and in order to determine the user compliance based on a piecewise function, a maximum TIB may be set. The maximum TIB is the maximum bedtime for which a person can sleep, for example, 20 hours.

The maximum TIB duration may be changed based on a defined minimum value (e.g., ten hours), and may be adjusted based on sleeping data of the user. Alternatively, the maximum TIB duration may be set to be greater than at least 2xR-TIB.

When the R-TIB is a and the maximum TIB duration is b, the user compliance curve may be determined based on the following equation.

[Equation 1-2]

$$y = \{x < a : -\frac{100}{a^2}(x - a)^2 + 100, \qquad x \geq a : -\frac{100}{(b - a)^2}(x - a)^2 + 100\ ]$$

Here, x denotes a TIB and y denotes user compliance. For example, when the R-TIB is 6 and the TIB is 4, the user compliance is 88.9%, and when the R-TIB is 6 and the TIB is 9, the user compliance is 97.9%.

The user compliance curve may be defined as a power of 2 as shown in Equation 1-2, or may be defined as a power of another even number providing a steeper curve. The number for an exponent for determining the user compliance curve may be adaptively changed according to the user compliance feedback value.

Referring to the user compliance curve, the R-TIB is generally less than half of the maximum TIB duration. Accordingly, the user compliance curve may be defined to determine the user compliance more strictly in a part having a sleep time less than the R-TIB compared to a part having a sleep time more than the R-TIB.

However, when the R-TIB is greater than half of the maximum TIB duration (e.g., R-TIB=6 and max TIB duration=10), the user compliance curve may have a steep slope such that the compliance of people who slept more than the R-TIB decreases relatively rapidly, as shown on the right side of the curve.

The user compliance curve may be generated based on the fact that sleeping less is less desirable than sleeping more in the treatment of insomnia.

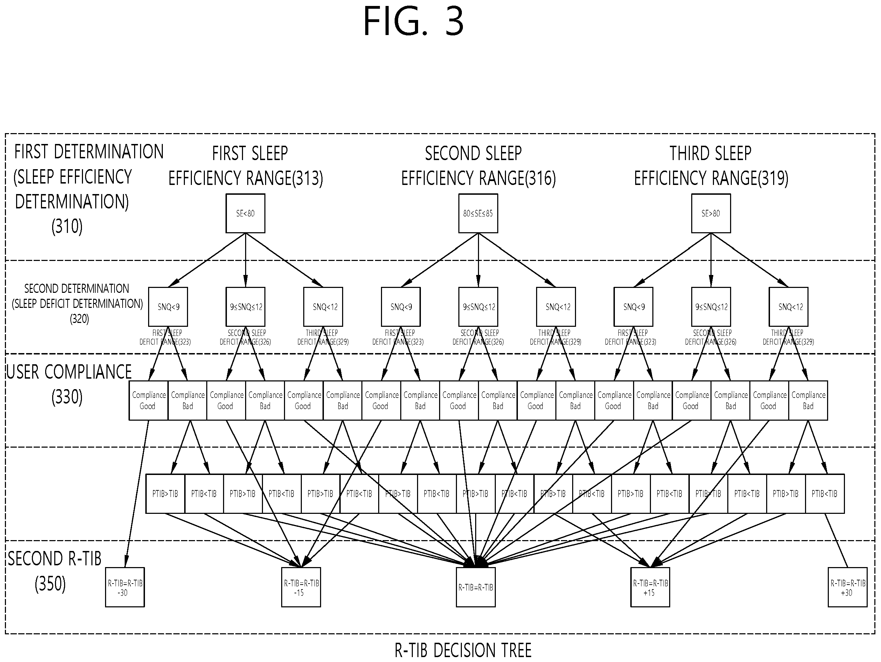

FIG. 3 is a conceptual diagram illustrating an operation of an R-TIB determiner according to an embodiment of the present invention.

In FIG. 3, a method of the R-TIB determiner 130 determining an R-TIB is disclosed.

Referring to FIG. 3, a method of the R-TIB determiner 130 determining an R-TIB by performing a first determination (a sleep efficiency determination) 310 on the sleep of the user, and performing a second determination (a sleep deficit determination) 320 on the sleep of the user is disclosed.

Among three sleep efficiency ranges set based on the first determination (the sleep efficiency determination) 310, one sleep efficiency range may be set as the sleep efficiency range of the user. Among three sleep deficit ranges set based on the second determination (the sleep deficit determination) 320, one sleep deficit range may be set as the sleep deficit range of the user.

The interval and the number of the range sections may be varied, and a method of varying the interval and the number of the range sections may also be included in the scope of the present invention. In addition, a first sleep efficiency range 313, a second sleep efficiency range 316, and a third sleep efficiency range 319, which are three sleep efficiency ranges set based on the first determination (the sleep efficiency determination) 310 in the present invention, and a first sleep deficit range 323, a second sleep deficit range 326, and a third sleep deficit range 329, which are three sleep deficit ranges set based on the second determination (the sleep deficit determination) 320 in the present invention are arbitrary and subject to change.

First, the first determination (the sleep efficiency determination) 310 may be performed on the sleep of the user.

The sleep efficiency of the user may be classified based on sleep efficiency (SE) determined by the sleep quality determiner 100, as one of the first sleep efficiency range (SE<80) 313, the second sleep efficiency range (80<=SE<=85) 316, and the third sleep efficiency range (SE>85) 319.

After the determination of the sleep efficiency range, the second determination (the sleep deficit determination) 320 may be performed on the sleep of the user.

The sleep deficit of the user may be classified based on a sleep need questionnaire (SNQ) determined by the sleep quality determiner 100 as one of the first sleep deficit range (SNQ<9) 323, the second sleep deficit range (9<=SNQ<=12) 326, and the third sleep deficit range (SNQ>12) 329.

After the first determination (the sleep efficiency determination) 310 and the second determination (the sleep deficit determination) 320, user compliance 330 may be determined. The user compliance 330 may be determined through a method of determining user compliance 330 based on the first R-TIB previously recommended to the user and the actual TIB of the user described above in FIG. 2.

When the user compliance 330 is greater than or equal to a threshold, the user compliance 330 may be determined to be in a first state (compliance good), and when the user compliance 330 is less than the threshold value, the user compliance 330 may be determined to be in a second state (compliance bad).

In response to the user compliance 330 being in the second state, a comparison in size between the first R-TIB and the TIB may be performed. It may be determined whether the user compliance 330 is in the second state because the first R-TIB is larger than the TIB, and conversely, whether the user compliance 330 is in the second state because the first R-TIB is smaller than the TIB.

A second R-TIB 350 may be determined.

The second R-TIB 350 may be determined based on the user sleep efficiency range, the user sleep deficit range, the user compliance, and the difference in size between the first R-TIB and the TIB.

Considering the sleep quality, the sleep time, and the first R-TIB and the TIB, option 1) determining the second R-TIB by decreasing the first R-TIB, option 2) determining the second R-TIB by maintaining the existing first R-TIB, or option 3) determining the second R-TIB by decreasing the first R-TIB may be determined. In addition, an increase amount of the first R-TIB and a decrease amount of the first R-TIB may also be determined in consideration of the sleep quality, the sleep time, the first R-TIB, and the TIB.

In FIG. 3, a rule for determining the second R-TIB in consideration of the sleep quality, the sleep time, the first R-TIB, and the TIB is illustrated.

Basically, the existing first R-TIB may be set to be maintained when the sleep efficiency is relatively high, the sleep deficit is relatively low, and the user compliance is relatively high, and adjustment of the first R-TIB may be performed in consideration of the sleep efficiency and the sleep deficit. Specifically, the adjustment of the first R-TIB may be set to decrease the first R-TIB as the sleep efficiency is lower, and may be set to increase the first R-TIB as the sleep efficiency is higher. In addition, the adjustment of the first R-TIB may be set to decrease the first R-TIB as the sleep deficit is relatively small, and may be set to increase the first R-TIB as the sleep deficit is relatively large.

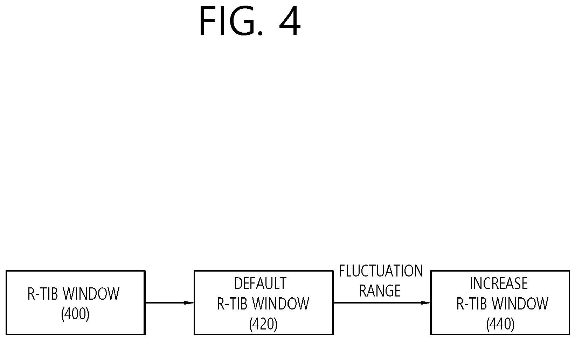

FIG. 4 is a conceptual diagram illustrating a method of extracting data for determining an R-TIB according to an embodiment of the present invention.

In FIG. 4, a method of setting an R-TIB window to determine an R-TIB with high accuracy and reliability is disclosed.

Referring to FIG. 4, an R-TIB window 400 may be basically set based on sleep data of the most recent n days (e.g., n=5), which is unmodifiable.

In order to increase the user's autonomy, sleep data of three days including the current date may be set to be directly edited and written by the user. Accordingly, the R-TIB window 400 may be basically determined based on sleep data of the most recent n days, which is unmodifiable. Such a setting of the R-TIB window 400 obviates a need to consider changes such as a case in which the sleep diary written by the user is modified and thus the R-TIB is repeatedly applied, so that the reliability of the R-TIB may be increased.

In order to select the R-TIB window 400, a fluctuation range may be checked. For example, there may be a large difference between a weekend sleep pattern and a weekday sleep pattern. In the case of weekends, the fluctuation range of sleep may be relatively large. When exceptional data is included as an outlier in the calculation of the R-TIB, a result that is inaccurate or unhelpful to the patient may be output.

The R-TIB window 400 may be initially set to a default R-TIB window (e.g., five days) 420, and a fluctuation range of the default R-TIB window 420 may be determined. The fluctuation range of the default R-TIB window 420 may be calculated based on a standard deviation. When the fluctuation range of the default R-TIB window 420 is greater than or equal to a threshold fluctuation range, an increase R-TIB window 440 of six days may be set by adding one more day to the original default R-TIB window 420. When the increase R-TIB window 440 is set, the fluctuation range may decrease as more is added to the sleep diary, and the R-TIB may be determined from sleep data of n' pieces of sleep data of the user included in the increase R-TIB window 400 based on the fluctuation range being less than the threshold fluctuation range. The R-TIB determined through the method of adjusting the R-TIB window may have improved reliability and accuracy.

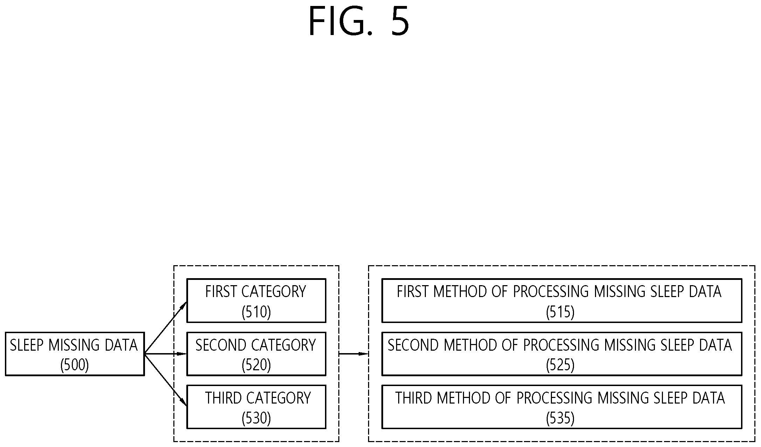

FIG. 5 is a conceptual diagram illustrating a method of processing missing data according to an embodiment of the present invention.

In FIG. 5, a method of determining an R-TIB through processing of missing sleep data that is missing in sleep data is disclosed.

Referring to FIG. 5, missing sleep data 500 may be classified into three categories.

A first category 510 is a case in which missing sleep data 500 is generated without a miss pattern.

A second category 520 is a case in which missing sleep data 500 is generated with a miss pattern.

A miss pattern is a regular occurrence of omission of sleep data, such as omission of sleep data at a specific time. For example, when missing sleep data is generated because sleep data is not written on the weekend, the missing sleep data is considered as having a miss pattern.

A third category 530 is a case in which missing sleep data 500 is generated with an intention to omit sleep data. For example, this is a case of omission of sleep data generation on a day when the user fails to sleep, to intentionally hide that the user fails to sleep.

In the present invention, the missing sleep data 500 may be processed in consideration of the three categories of missing sleep data in various ways to determine the R-TIB.

As a first method 515 of processing missing sleep data, a likewise deletion method may be used. The likewise deletion method is a method of determining the R-TIB by excluding ungenerated sleep data regardless of the category.

A second method 525 of processing missing sleep data is a method of determining the R-TIB by excluding missing sleep data 500 corresponding to the second category 520, and estimating missing sleep data 500 corresponding to the first category 510 and the third category 530 to generate sleep data. The missing sleep data 500 of the first category 510 may be generated through estimation based on sleep data of nearby dates, that is, sleep data of a previous date and a following date. The missing sleep data 500 of the third category 530 may be generated through recommendation based on sleep data of a date having the lowest sleep efficiency and the highest sleep deficit in the R-TIB window.

A third method 535 of processing missing sleep data is a method of determining the R-TIB by excluding missing sleep data 500 corresponding to the first category 510 and the second category 520, and by estimating only missing sleep data corresponding to the third category 530 to generate sleep data.

The first method 515 of processing missing sleep data, the second method 525 of processing missing sleep data, and the third method 535 of processing missing sleep data may be selectively used according to a user.

In consideration of the existing user compliance of the user, the first method 515 of processing missing sleep data may be used when the user compliance is greater than or equal to a first threshold value.

In consideration of the existing user compliance of the user, the second method 525 of processing missing sleep data may be used when the user compliance is greater than or equal to a second threshold value and less than the first threshold value.

In consideration of the existing user compliance of the user, the third method 535 of processing missing sleep data may be used when the user compliance is less than the second threshold value.

That is, considering whether the existing user has complied with the method of treating a sleep disorder based on data according to the embodiment of the present invention, the R-TIB may be determined based on a stricter criterion as the user compliance is lower.

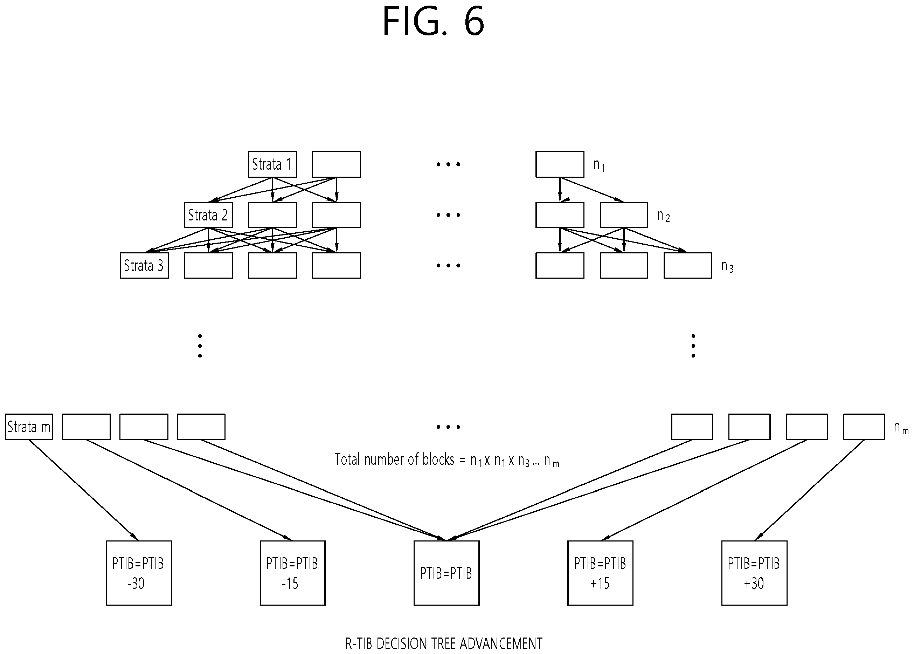

FIG. 6 is a conceptual diagram illustrating a method of determining an R-TIB according to an embodiment of the present invention.

In FIG. 6, a method of advancing the determination of the R-TIB by further specifying sleep efficiency ranges and sleep deficit ranges is illustrated.

Referring to FIG. 6, the sleep efficiency ranges and the sleep deficit ranges in the R-TIB determination method disclosed in FIG. 3 are divided in more detail so that an R-TIB decision tree may be advanced.

Through an increase in the number of sleep efficiency range sections, in which sleep efficiency range sections are divided into a larger number of sections, and/or an increase in the number of sleep deficit range sections, in which sleep deficit range sections are divided into a larger number of sections, the R-TIB may be determined through a more detailed classification.

The R-TIB may be determined based on a stricter criterion as user compliance is lower. Therefore, as the user compliance is lower, the R-TIB may be more precisely determined through an increase in the number of the sleep efficiency range sections and an increase in the number of the sleep deficit range sections. Conversely, as the user compliance is higher, the R-TIB may be determined through a decrease in the sleep efficiency range sections and a decrease in the number of the sleep deficit range sections.

The embodiments of the present invention can be implemented in the form of program commands executable by a variety of computer components and may be recorded on a computer readable medium. The computer readable medium may include, alone or in combination, program commands, data files and data structures. The program commands recorded on the computer readable medium may be components specially designed for the present invention or may be usable to a skilled person in the field of computer software. Computer readable record media include magnetic media such as a hard disk, a floppy disk, or a magnetic tape, optical media such as a compact disc read only memory (CD-ROM) or a digital video disc (DVD), magneto-optical media such as floptical disks, and hardware devices such as a ROM, a random-access memory (RAM), or a flash memory specially designed to store and carry out programs. The program commands include not only a machine language code made by a compiler but also a high-level code that can be used by an interpreter etc., which is executed by a computer. The hardware device may be configured to act as one or more software modules in order to perform the operations of the present invention, or vice versa.

As is apparent from the above, a sleep disorder of a user can be treated by recommending a more therapeutically effective bedtime in consideration of the sleep efficiency of the user using data.

A sleep disorder of a user can be treated by appropriately adjusting a recommended bedtime of the user to control the balance between the patient's compliance and therapeutic performance.

A sleep disorder of a user can be treated through adaptive management of sleep data and generation of an R-TIB tree in consideration of the user compliance of the user.

The effects of the present application are not limited to the above-described effects, and effects not described may be clearly understood by those of ordinary skill in the art from the present specification and the accompanying drawings.

While the invention has been shown and described with respect to particulars, such as specific components, embodiments, and drawings, the embodiments are used to aid in the understanding of the present invention rather than limiting the present invention, and those skilled in the art should appreciate that various changes and modifications are possible without departing from the spirit and scope of the invention.

Therefore, the spirit of the present invention is not defined by the above embodiments but by the appended claims of the present invention, and the scope of the present invention is to cover not only the following claims but also all modifications and equivalents derived from the claims.

**Claims**

1. A method of treating a sleep disorder based on data, the method comprising:

    determining, by a sleep disorder treatment apparatus, sleep quality;
    determining, by the sleep disorder treatment apparatus, user compliance with a first recommended time in bed (R-TIB); and

determining, by the sleep disorder treatment apparatus, a second R-TIB on the basis of the sleep quality and the user compliance,

wherein the first R-TIB is a sleep time recommended to a user earlier, and

the second R-TIB is a sleep time recommended to the user later.

2. The method of claim 1, wherein the sleep quality includes sleep efficiency and a sleep deficit, and the user compliance is determined based on slopes that are set to be different according to ranges of a time in bed (TIB) or determined based on a user compliance curve.

3. The method of claim 2, wherein the second R-TIB is determined based on a user sleep efficiency range, a user sleep deficit range, the user compliance, and a difference in size between the first R-TIB and the TIB,

the user sleep efficiency range is a sleep efficiency range of the user among a (here, a is a natural number) set sleep efficiency ranges based on a first determination on the sleep efficiency (a sleep efficiency determination),

the user sleep deficit range is a user sleep deficit range of the user among b (here, b is a natural number) set sleep deficit ranges based on a second determination on the sleep deficit (a sleep deficit determination),

the user sleep deficit range is determined, after determination of the user sleep efficiency range, based on the determined user sleep efficiency range, and

the second R-TIB is determined based on a default R-TIB window or an increase R-TIB window determined in consideration of a sleep fluctuation range,

wherein the increase R-TIB window is determined such that the sleep fluctuation range is less than a threshold fluctuation range.

4. A sleep disorder treatment apparatus for performing sleep disorder treatment based on data, the sleep disorder treatment apparatus comprising:

a sleep quality determiner configured to determine sleep quality;

a user compliance determiner configured to determine user compliance with a first recommended time in bed (R-TIB); and

an R-TIB determiner configured to determine a second R-TIB on the basis of the sleep quality and the user compliance,

wherein the first R-TIB is a sleep time recommended to a user earlier, and

the second R-TIB is a sleep time recommended to the user later.

5. The sleep disorder treatment apparatus of claim 4, wherein the sleep quality includes sleep efficiency and a sleep deficit, and

the user compliance is determined based on slopes that are set to be different according to ranges of a time in bed (TIB) or determined based on a user compliance curve.

6. The sleep disorder treatment apparatus of claim 5, wherein the second R-TIB is determined based on a user sleep efficiency range, a user sleep deficit range, the user compliance, and a difference in size between the first R-TIB and the TIB,

the user sleep efficiency range is a sleep efficiency range of the user among a (here, a is a natural number) set sleep efficiency ranges based on a first determination on the sleep efficiency (a sleep efficiency determination),

the user sleep deficit range is a user sleep deficit range of the user among b (here, b is a natural number) set sleep deficit ranges based on a second determination on the sleep deficit (a sleep deficit determination),

the user sleep deficit range is determined, after determination of the user sleep efficiency range, based on the determined user sleep efficiency range, and

the second R-TIB is determined based on a default R-TIB window or an increase R-TIB window determined in consideration of a sleep fluctuation range,

wherein the increase R-TIB window is determined such that the sleep fluctuation range is less than a threshold fluctuation range.

# FIG. 1

SLEEP DISORDER
TREATMENT APPARATUS

SLEEP QUALITY DETERMINER
(100)

SLEEP TIME DETERMINER
(110)

USER COMPLIANCE DETERMINER
(120)

R-TIB DETERMINER
(130)

PROCESSOR
(140)

# FIG. 2A

# FIG. 2B

# FIG. 3

R-TIB DECISION TREE

EP 4 195 213 A1

# FIG. 4

```
┌─────────────────┐      ┌─────────────────┐  FLUCTUATION  ┌─────────────────┐
│  R-TIB WINDOW   │─────▶│     DEFAULT     │    RANGE      │    INCREASE     │
│     (400)       │      │  R-TIB WINDOW   │──────────────▶│  R-TIB WINDOW   │
│                 │      │     (420)       │               │     (440)       │
└─────────────────┘      └─────────────────┘               └─────────────────┘
```

# FIG. 5

```
                          ┌─────────────────────┐   ┌──────────────────────────────────────────────┐
                          │ ┌─────────────────┐ │   │ ┌──────────────────────────────────────────┐ │
                          │ │ FIRST CATEGORY  │ │   │ │ FIRST METHOD OF PROCESSING MISSING SLEEP DATA│ │
                          │ │     (510)       │ │   │ │                  (515)                     │ │
                          │ └─────────────────┘ │   │ └──────────────────────────────────────────┘ │
  ┌──────────────────┐    │ ┌─────────────────┐ │   │ ┌──────────────────────────────────────────┐ │
  │ SLEEP MISSING DATA│──▶│ │ SECOND CATEGORY │ │──▶│ │SECOND METHOD OF PROCESSING MISSING SLEEP DATA│ │
  │     (500)         │    │ │     (520)       │ │   │ │                  (525)                     │ │
  └──────────────────┘    │ └─────────────────┘ │   │ └──────────────────────────────────────────┘ │
                          │ ┌─────────────────┐ │   │ ┌──────────────────────────────────────────┐ │
                          │ │ THIRD CATEGORY  │ │   │ │ THIRD METHOD OF PROCESSING MISSING SLEEP DATA│ │
                          │ │     (530)       │ │   │ │                  (535)                     │ │
                          │ └─────────────────┘ │   │ └──────────────────────────────────────────┘ │
                          └─────────────────────┘   └──────────────────────────────────────────────┘
```

# FIG. 6

EP 4 195 213 A1

Strata 1 — $n_1$

Strata 2 — $n_2$

Strata 3 — $n_3$

Strata m — $n_m$

Total number of blocks = $n_1$ x $n_1$ x $n_3$ ... $n_m$

| PTIB=PTIB -30 | PTIB=PTIB -15 | PTIB=PTIB | PTIB=PTIB +15 | PTIB=PTIB +30 |

R-TIB DECISION TREE ADVANCEMENT

# EP 4 195 213 A1

## INTERNATIONAL SEARCH REPORT

International application No.

**PCT/KR2022/003881**

| A. | CLASSIFICATION OF SUBJECT MATTER |
|---|---|
| | **G16H 20/00**(2018.01)i; **G16H 10/60**(2018.01)i; **G16H 50/20**(2018.01)i; **A61B 5/00**(2006.01)i |

According to International Patent Classification (IPC) or to both national classification and IPC

| B. | FIELDS SEARCHED |
|---|---|

Minimum documentation searched (classification system followed by classification symbols)

G16H 20/00(2018.01); A61B 5/16(2006.01); A61M 21/00(2006.01); G06Q 50/22(2012.01); G16H 20/70(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 수면 장애 치료(treating sleep disorder), 수면질(sleep quality), R-TIB(recommended time in bed), 사용자 순응도(user compliance)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | KR 10-2019-0092765 A (PARK, Sung Kyu et al.) 08 August 2019 (2019-08-08)<br>See paragraphs [0040]-[0058]; and figures 1-4. | 1-6 |
| A | KR 10-2020-0097421 A (PARK, Sung Kyu et al.) 19 August 2020 (2020-08-19)<br>See paragraphs [0033]-[0051]; and figures 1-4. | 1-6 |
| A | JP 2021-135853 A (SEKISUI CHEM. CO., LTD.) 13 September 2021 (2021-09-13)<br>See claim 1; and figure 2. | 1-6 |
| A | JP 2016-122347 A (OMRON CORP.) 07 July 2016 (2016-07-07)<br>See claims 1-3; and figure 1. | 1-6 |
| A | JP 2017-046960 A (TDK CORP.) 09 March 2017 (2017-03-09)<br>See claims 1-8; and figures 4 and 6-7. | 1-6 |

☐ Further documents are listed in the continuation of Box C.  ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **15 July 2022** | **19 July 2022** |

| Name and mailing address of the ISA/KR | Authorized officer |
|---|---|
| **Korean Intellectual Property Office**<br>**Government Complex-Daejeon Building 4, 189 Cheongsa-**<br>**ro, Seo-gu, Daejeon 35208** | |
| Facsimile No. **+82-42-481-8578** | Telephone No. |

Form PCT/ISA/210 (second sheet) (July 2019)

**17**

**INTERNATIONAL SEARCH REPORT**
Information on patent family members

International application No.

**PCT/KR2022/003881**

| Patent document cited in search report | | | Publication date (day/month/year) | Patent family member(s) | | | Publication date (day/month/year) |
|---|---|---|---|---|---|---|---|
| KR | 10-2019-0092765 | A | 08 August 2019 | KR | 10-2141804 | B1 | 06 August 2020 |
| KR | 10-2020-0097421 | A | 19 August 2020 | None | | | |
| JP | 2021-135853 | A | 13 September 2021 | None | | | |
| JP | 2016-122347 | A | 07 July 2016 | CN | 107111852 | A | 29 August 2017 |
| | | | | CN | 107111852 | B | 09 October 2020 |
| | | | | EP | 3211589 | A1 | 30 August 2017 |
| | | | | JP | 6485037 | B2 | 20 March 2019 |
| | | | | US | 11004551 | B2 | 11 May 2021 |
| | | | | US | 2017-0312477 | A1 | 02 November 2017 |
| | | | | WO | 2016-104325 | A1 | 30 June 2016 |
| JP | 2017-046960 | A | 09 March 2017 | JP | 6657682 | B2 | 04 March 2020 |

Form PCT/ISA/210 (patent family annex) (July 2019)

**REFERENCES CITED IN THE DESCRIPTION**

**Patent documents cited in the description**

- KR 20210141738